# EUROPÄISCHE PATENTSCHRIFT

(11) **EP 1 101 506 B1**
(45) Veröffentlichungstag und Bekanntmachung des Hinweises auf die Patenterteilung: **07.04.2004**
(21) Anmeldenummer: 00250382.9
(22) Anmeldetag: 16.11.2000
(51) Int. Cl.: A61M 13/00

(54) **Vorrichtung zum Insufflieren von Gas**
Gas insufflation device
Dispositif pour insuffler un gaz

(30) Priorität: 17.11.1999 DE 19955847
(43) Veröffentlichungstag der Anmeldung: 23.05.2001
(73) Patentinhaber: W.O.M. World of Medicine AG, 96337 Ludwigsstadt (DE)
(72) Erfinder: Schramm, Eckhard, 25451 Quickborn (DE); Mehner, Gotthilf, 66280 Sulzbach (DE)
(74) Vertreter: Lüke, Dierck-Wilm

(56) Entgegenhaltungen:
- WO-A-94/01154
- DE-A- 19 510 710
- DE-A- 19 822 751
- US-A- 5 061 268
- US-A- 5 354 268
- US-A- 5 383 874

## Beschreibung

Die Erfindung betrifft eine Vorrichtung zum Insufflieren von Gas in einen menschlichen oder tierischen Körper mittels eines über einen Schlauch an eine Gasversorgungseinrichtung anschließbaren Insufflationsinstruments, wobei der Schlauch mit der Gasversorgungseinrichtung mittels einer Schlauchanschlußschnittstelle, aufweisend zueinander komplementäre Verbindungselemente an oder in der Gasversorgungseinrichtung sowie an dem gasversorgungsseitigen Ende des Schlauches, verbindbar ist, und wobei ein Sterilfilterelement eingerichtet ist. - Vorrichtungen dieser Art werden insbesondere im Bereich der Endoskopie bzw. Laparoskopie verwendet. Die typischerweise eingesetzte Gasart ist Kohlendioxid, wobei selbstverständlich aber auch andere Gase, ggf. mit pharmazeutischen Zusatzstoffen, verwendet werden können. Als Insufflationsinstrument kommt bspw. eine Veress-Nadel in Frage. Eine Gasversorgungseinrichtung umfaßt in der Regel eine Gasquelle bzw. einen Anschluß an eine Gasquelle, einen Druckminderer, einen Durchflußmesser und/oder Druckmesser sowie eine Steuerschaltung zur Druck- bzw. Durchflußsteuerung. Es versteht sich, daß die genannten Bauelemente in geeigneter Weise über Gasleitungen (Schläuche oder Rohre) miteinander verbunden sind. Die vorstehend angesprochenen Elemente einer Gasversorgungseinrichtung sind jedoch nicht Gegenstand der vorliegenden Erfindung und fur eine lediglich bspw. konkrete Ausführungsform wird daher auf die Literaturstelle DE 3611018 C1 verwiesen.

Bei Vorrichtungen des eingangs genannten Aufbaus stellt sich in der Praxis das Problem hinreichender Sterilität. Grundsatzlich ist es wünschenswert, daß alle mit dem Gas in Berührung kommende Bauteile, Bauelemente und Instrumente vor dem Eingriff steril sind bzw. sterilisiert werden können. Hinsichtlich der Sterilität ergeben sich jedoch zwei Problemkreise. Ein erster Problemkreis besteht darin, daß Schlauchverbindungen und Rohrleitungen innerhalb einer Gasversorgungseinrichtung nicht ohne weiteres vor einer Verkeimung geschützt werden können. Dies gilt insbesondere auch für den Produktionsprozeß. Es ist folglich damit zu rechnen, daß bereits neu ausgelieferte Gasversorgungseinrichtungen intern eine Keimbesiedelung aufweisen, welche sich mit zunehmendem Gebrauch ausbreitet. Der zweite Problemkreis liegt im Einsatzbereich der Vorrichtung. Trotz Gasfluß in Richtung Patienten ist eine Kontamination von Bauelementen der Gasversorgungseinrichtung mit Keimen entgegen der Gasflußrichtung nicht ausgeschlossen, unter bestimmten Bedingungen sogar wahrscheinlich. Mittels der Vorrichtung wird nämlich in einer Körperhöhle, in welche das Gas eingeleitet wird, ein Überdruck erzeugt. Durch besondere Umstände, z.B. durch manuellen Druck auf das Abdomen, kann es dann zu einem Reflux in die Gasversorgungseinrichtung kommen. Aerosole mit Korperflussigkeit bzw. Korperflussigkeit selbst konnen somit Eintritt in das Insufflationsinstrument, den daran angeschlossenen Schlauch bis in die Gasversorgungseinrichtung finden. Bei Einsatz an einem weiteren Patienten besteht dann, auch bei Austausch des Insufflationsinstruments sowie des Schlauches, die Gefahr der Kreuzkontamination.

Zur Losung dieser Problematik ist es aus der Praxis bekannt, einen Sterilfilter zwischen Insufflationsinstrument und Gasversorgungseinrichtung einzusetzen. Der Sterilfilter wird dabei in den Schlauch, welcher zwischen der Gasversorgungseinrichtung und dem Insufflationsinstrument eingerichtet ist, eingefügt. Dies ist relativ umständlich, und macht den Schlauch zudem unhandlich. Weiterhin kann im Rahmen der insofern bekannten Vorrichtung nicht mit hinreichender Sicherheit ausgeschlossen werden, daß eine Bedienperson gar kein Sterilfilter oder ein gebrauchtes Sterilfilter einsetzt mit der Folge einer gesundheitlichen Gefährdung des Patienten.

Aus der Druckschrift WO 94/01154 ist eine Vorrichtung zum Insufflieren von Gas bekannt, bei der ein Filter dazu eingesetzt wird, um unerwünschte Stoffe aus dem Hochdruckbehälter bzw. bakterielle oder virale Stoffe des menschlichen Körpers aus dem Gas zu entfernen. Der Filter ist verbunden mit der Gasversorgungseinrichtung und mit dem Insufflationsschlauch.

Die Druckschrift US 5.354,268 beschreibt eine Einrichtung zur Druckbeaufschlagung. Die Einrichtung besitzt einen Ausgang, an dem ein chirurgisches Instrument angeschlossen werden kann. Die gasführende Ausgangsleitung ist an einem mikrobiologischen Filter angeschlossen, wobei dieser wiederum über eine gefilterte gasführende Leitung mit dem chirurgischen Instrument verbunden ist.

In der Druckschrift US 5.061,268 wird ein System mit einem Koaggulations-Stift beschrieben, bei dem ein abnehmbarer Filter an einem ersten Ende an die Gasversorgung und an einem zweiten Ende an eine Gasleitung angeschlossen wird. Der Filter verhindert, dass Verunreinigungen wie Bakterien durch den Kontakt des Patienten mit dem Koaggulations-Stift, der über eine Zufuhr von inertem Gas verfügt, übertragen werden.

Ein System zur Identifizierung und Authentifizierung von Zubehör, Hilfs- und/oder Betriebsstoffen für technische Geräte ist aus der Druckschrift DE 198 22751 A1 bekannt. Zur Identifizierung einer Authorisierung des Zubehörs, der Hilfs- und oder/Betriebsstoffe weist das Zubehör einer zweistelligen Codierung auf, deren Analyse und Vergleich mit im technischen Gerät gespeicherten Informationen Auskunft über die ordnungsgemäße Authorisierung durch den Hersteller gibt.

Demgegenüber liegt der Erfindung das technische Problem zugrunde, eine Vorrichtung zum Insufflieren von Gas anzugeben, mit welcher einerseits ein steriles Arbeiten stets sicher gestellt ist und welcher andererseits einfach handhabbar ist. Desweiteren soll sichergestellt werden, daß eine absichtliche oder missbräuchliche Wiederverwendung bereits gebrauchter Sterilfilterelemente verhindert wird.

Zur Lösung dieses technischen Problems lehrt die Erfindung, daß das Sterilfilterelement das Verbindungselement des Schlauches trägt. - Mit anderen Worten ausgedrückt, das Sterilfilterelement wird nicht mehr auf umständliche Weise in der Schlauchleitung eingesetzt, sondern erhält eine Doppelfunktion, nämlich einerseits die Einrichtung einer Sterilbarriere und andererseits als mechanisches Verbindungselement zum Anschluß des Instruments bzw. dessen Schlauches an die Gasversorgungseinrichtung. Es versteht sich, daß das Verbindungselement der Gasversorgungseinrichtung so ausgebildet ist, daß nicht mittels lediglich eines Schlauchendes eine Verbindung hergestellt werden kann. Dies führt einerseits zu einer besonders einfachen Handhabung. Andererseits wird als besonders wichtiger Vorteil erreicht, daß ein Gebrauch der Vorrichtung ohne Sterilfilterelement, sei es absichtlich oder sei es versehentlich, praktisch nicht moglich ist. Denn ein Anschluß des Insufflationsinstruments bzw. des Schlauches an die Gasversorgungseinrichtung gelingt nur über das Sterilfilterelement. Wenn das Sterilfilterelement fest verbunden ist mit dem Schlauch, ist auch gewährleistet, daß mit einem Verwerfen des Sterilfilterelements auch gleichzeitig der hiermit verbundene Schlauch verworfen wird, und umgekehrt. Zudem konnen Sterilfilterelement und Schlauch als Einheit steril abgepackt und bis zur Verwendung aufbewahrt werden. Der Begriff der festen Verbindung meint in diesem Zusammenhang, daß der Schlauch nicht manuell von dem Sterilfilterelement abgezogen werden kann.

Der Aufbau kann im einzelnen der Gestalt sein, daß das Sterilfilterelement ein Filtergehäuse sowie eine darin angeordnete Filtereinheit aufweist, wobei ein Anschlußbereich des Filtergehäuses als Verbindungselement ausgebildet ist. Bei der Einrichtung und Anordnung der Filtereinheit ist selbstverständlich zu beachten, daß die Filtereinheit von dem Gas vollständig durchströmt wird und keine Nebenpfade bzw. Leckstellen für das strömende Gas bestehen bleiben. Das Filterelement kann bspw. als Membranfilter mit einer Durchflußleistung von 5-100 l/min., vorzugsweise von 16-60 l/min, ausgebildet sein. Als Material kommt bspw. PIFE mit einer Porengroße von 1-5 µm, vorzugsweise ca. 3 um, in Frage. Solche Membranfilter haben eine Filtrationseffizienz von 99,9998%. Grundsatzlich sind jedoch auch alle anderen, eine hinreichende Heimretention bewirkende Filterelementarten mit ausreichend niedrigem Gasstromungswiderstand einsetzbar.

Die beiden zueinander komplementaren Verbindungselemente können grundsätzlich auf die verschiedenste Weise ausgeführt sein. So sind bspw. Bajonettverbindungen, Schraub- und/oder Steckverbindungen oder in der Schlauch- bzw. Leitungstechnologie übliche Schnellkupplungen geeignet. In einer bevorzugten Ausführungsform der Erfindung weisen die Verbindungselemente zueinander komplementäre Schwalbenschwanzelemente, welche ineinander einschiebbar sind, auf. Die Schwalbenschwanzelemente können hinsichtlich ihrer Längserstreckung in einer zur Strömungsrichtung des Gases im wesentlichen orthogonalen Fläche eingerichtet sein; es ist auch möglich, die Schwalbenschwanzelemente in einer einfach oder doppelt gekrümmten Fläche (z.B. Zylindermantelteilfläche oder sphärische Teilfläche) verlaufen zu lassen. Bei dieser Ausführungsform wird zum Anschluß des Schlauches das am Sterilfilterelement angeordnete Verbindungselement in das hierzu komplementäre Verbindungselement an der Gasversorgungseinrichtung eingeschoben und bei vollständigem Einschub stehen ein Lumen der Gasversorgungseinrichtung und ein Lumen des Sterilfilterelements einander gegenüber (gegen die Umgebung abgedichtet) und erlauben den Gasdurchfluß. In diesem Zusammenhang kann eines der Verbindungselemente ein Betätigungselement fur einen in der Gasversorgungseinrichtung eingerichteten Verschlußschieber aufweisen, welcher bei nicht angeschlossenem Verbindungselement des Schlauches die Gasversorgungseinrichtung, beispielsweise das Geratelumen im Bereich des Verbindungselementes der Gasversorgungseinrichtung, gasdicht abschließt. Das Betatigungselement kann eine Kante am Sterilfilterelement sein, welche den Verschlußschieber beim Einschieben des Sterilfilterelements vor sich herschiebt und so aus einer Geschlossenstellung in eine Offenstellung verschiebt oder verschwenkt. Der VerschlußSchieber kann dabei in Richtung auf seine Geschlossenstellung federkraftbeaufschlagt sein. Hierbei kann in der Offenstellung des Verschlußschiebers zusätzlich ein Rast- bzw. Halteelement eingerichtet sein, welches den Verschlußschieber gegen die Federkraft in der Offenstellung hält. Dies kann bspw. in Form einer (mechanisch oder elektromechanisch lösbaren) Sperrklinke vorgesehen sein. Dann kann im Rahmen der Gasversorgungseinrichtung ein Betätigungselement vorgesehen sein, mit welchem diese Sperrklinke gelöst wird und - bei ausreichender Federkraftbeaufschlagung auf den Verschlußschieber - das Sterilfilterelement mittels des Verschlußschiebers wieder auswirft. Es versteht sich, daß auch andere Steuerungen des Verschlußschiebers möglich sind, bspw. mittels elektromotorischer Antriebe. Insofern kann das Betätigungselement an einem der Verbindungselemente auch anders als mechanisch wirken. Die vorstehend im Zusammenhang mit den komplementären Schwalbenschwanzelemente angesprochene Verschlußschiebertechnologie läßt sich grundsätzlich selbstverständlich auch auf andere Ausführungsformen der Verbindungselemente anpassen und übertragen, was mit den konstruktiven Kenntnissen des Durchschnittsfachmanns unschwer gelingt.

Erfindungsgemäß ist weiterhin vorgesehen, daß das Sterilfilterelement ein maschinenlesbares Identelement aufweist, wobei das Identelement mit der Maßgabe angeordnet ist, daß es bei miteinander verbundenen Verbindungselementen mittels einer im Rahmen der Gasversorgungseinrichtung eingerichteten und mit einer Auswerteeinheit verbundene Leseeinheit lesbar ist. Als Identelement wird ein Merkmal bezeichnet, welches Informationen bezüglich des Sterilfilterelements enthält. Maschinenlesbare Identelemente sind in den verschiedensten Ausführungsformen bekannt und im Rahmen der Erfindung einsetzbar. Beispiele hierfür sind: Barcode, Magnetstreifen, Microchip und Hologramm. Im Falle des Magnetstreifens sowie des Microchips ist es auch möglich, den Informationsgehalt des Identelements zu verändern und/oder zu ergänzen, wobei dann die Leseeinheit zusätzlich in geeigneter Weise als Schreibeinheit ausgebildet sein muß oder eine zusätzliche Schreibeinheit eingerichtet sein muß. Im Falle des Barcodes ist die Leseeinheit ein optischer Scanner. Im Falle des Magnetstreifens ist die Leseeinheit ein Magnetlesekopf, welcher ggf. auch gleichzeitig als Schreibkopf genutzt werden kann. Im Falle des Mikrochips ist die Leseeinheit ein Kontaktfeld, welches zugeordnete Kontaktfelder des Identelements bei verbundenen Verbindungselementen kontaktiert. Im Falle des Hologramms umfaßt die Leseeinheit eine Quelle kohärenten Lichts sowie eine geeignete optische Sensorik. In jedem Fall findet eine geeignete Umwandlung der von der Leseeinheit aufgenommenen Eigenschaften in elektrische Signale statt, welche, ggf. nach A/D-Wandlung der vorzugsweise digitalen Auswerteeinheit als Informationssignale zugeführt werden. In der Auswerteeinheit wird die mittels der Leseeinheit aus dem Identelement ausgelesene Information ausgewertet und nach Maßgabe des Ergebnisses dieser Auswertung die Gasversorgungseinrichtung freigeschaltet oder gesperrt. Hierzu umfaßt die Auswerteeinheit Speicherelemente, in welchen die Information sowie Referenzinformation abspeicherbar bzw. permanent abgespeichert sind. Hierdurch läßt sich eine absichtliche oder mißbräuchliche Wiederverwendung bereits gebrauchter Sterilfilterelemente zuverlässig durch geeignete Maßnahmen im Rahmen der Auswerteeinheit verhindern. So ist es bspw. möglich, daß ein als Barcode ausgeführtes Identelement ein für jedes Sterilfilterelement verschiedenes Codeelement (beispielsweise eine fortlaufende Numerierung codierend) aufseist. Bei der Erstverwendung eines Sterilfilterelements wird dann das Codeelement eingelesen und im Rahmen der Auswerteeinheit als "aktuell" gespeichert und anhand entsprechend zuvor als "verbraucht" gespeicherter Codeelemente durch Vergleich überprüft, ob das aktuelle individuelle Codeelement zuvor bereits einmal eingelesen wurde. Im Falle des tatsächlichen Ersteinsatzes ist dies nicht der Fall und die Gasversorgungseinrichtung wird freigeschaltet. Die Information des aktuellen Codeelements wird dann (z.B. nach Auswurf des Filterelements) als "verbraucht" abgespeichert. Wenn dagegen das individuelle Codeelement vorher bereits einmal eingelesen wurde, i.e. bereits als "verbraucht" abgespeichert ist, so handelt es sich nicht mehr um eine Erstverwendung des betreffenden Sterilfilterelements und die Auswerteeinheit sperrt dann die Gasversorgungseinrichtung und gibt ggf. akustische oder optische Signale. Auch ist es möglich, daß das Sterilfilterelement dann automatisch ausgeworfen wird, z.B. durch Ansteuerung einer elektromechanisch betatigbaren Sperrklinke für den Verschlußschieber. Wenn z.B. mit einem Magnetstreifen und/oder einem Microchip gearbeitet wird, so kann im Pahmen einer Erstbenutzung alternativ oder zusätzlich ein Sperrcode in das Identelement geschrieben werden. Wenn dann eine Wiederverwendung des Sterilfilterelements versucht wird, so identifiziert die Auswerteeinheit den mittels der Leseeinheit eingelesenen Sperrcode mit der bereits zuvor geschilderten Folge eines Alarmsignals und/oder eines Filterelementauswurfes. In dieser Ausfuhrungsform ist sichergestellt, daß auch eine Wiederverwendung eines Sterilfilterelements an verschiedenen Geräten nicht durchgeführt werden kann.

Insgesamt wird mit der Ausfuhrungsform eines maschinenlesbaren Identelements eine sehr hohe Betriebssicherheit der erfindungsgemäßen Vorrichtung gewährleistet. Denn es bleibt nicht mehr einer Bedienperson überlassen, ob uberhaupt ein Sterilfilterelement eingesetzt wird und/oder ob ein bereits benutztes Sterilfilterelement nochmals verwendet werden könnte. Die erfindungsgemäße Vorrichtung nimmt den ordnungsgemaßen Betrieb nämlich nur dann auf, wenn ein neues, i.e. ungebrauchtes, Sterilfilterelement angeschlossen worden ist.

In einer vorteilhaften Weiterbildung der Erfindung umfaßt das Identelement ein Sicherheitselement. Ein Sicherheitselement meint ein manuell, visuell oder maschinenlesbares Element, welches mit einer hohen Fälschungssicherheit ausgestattet ist. Sicherheitselemente, auch maschinenlesbare Sicherheitselemente, sind bspw. aus der Technologie der Wertpapiere und Zahlungsmittel mannigfaltig bekannt und können in entsprechender Weise im Rahmen der vorliegenden Erfindung eingesetzt werden. Ein maschinenlesbares Sicherheitselement ist bspw. durch ein Hologramm gebildet. Hologramme lassen sich nur unter vergleichsweise sehr hohem technologischem Aufwand nachahmen, so daß eine Anbringung von gefälschten Hologrammen im Rahmen der Erfindung wirtschaftlich unattraktiv wird. Im Falle von maschinenlesbaren Sicherheitselementen (neben Hologrammen kommen hierfur bspw. auch magnetische Sicherheitselemente usw. in Frage) kann eine automatische Sperre der Gasversorgungseinrichtung bei Fehlen des Sicherheitselements und/oder den Anforderungen nicht genügendem Sicherheitselement durch die Auswerteeinheit bewirkt werden, wenn die Leseeinheit zum Lesen des Sicherheitselements eingerichtet ist und/oder eine Sicherheitselementleseeinrichtung zusätzlich vorgesehen ist. Mit einem selchen Sicherheitselement wird eine beachtlich erhöhte Sicherheit für Patienten erreicht, da Sterilfilterelemente von nicht authorisierten Herstellern, insbesondere von mit minderer Qualität arbeitenden Billiganbietern, von der Auswerteeinheit nicht akzeptiert werden. Eine Bedienperson kann folglich ausschließlich Sterilfilterelemente von den qualitätsüberwachten authorisierten Herstellern einlegen um die Gasversorgungsvorrichtung in Betrieb zu nehmen. Ein Sicherheitselement kann auch im Rahmen des Identelements als verschlüsseltes Sicherheitscodeelement eingerichtet sein.

In einer weiteren Ausführungsform der Erfindung kann im Sterilfilterelement abströmungsseitig der Filtereinheit ein Feuchtigkeitssensor angeordnet sein, welcher bei miteinander verbundenen Verbindungselementen zur Kommunikation mit der Auswerteeinheit eingerichtet ist. Diese Ausführungsform hat den folgenden Hintergrund. Zwar sind bzw. konnen Filtereinheiten hydrophob und insofern flüssigkeitssperrend ausgebildet sein. Diese hydrophoben Materialeigenschaften sind allerdings von den Herstellern nur uber bestimmte Zeitraume garantiert. Aus Sicherheitsgründen ist es daher empfehlenswert, wenn eine mit Flussigkeit

In der hier beschriebenen Ausfuhrungsform der Erfindung dient zur Detektion eines Vordringens von Flussigkeit bzw. Aerosol der vorzugsweise nahe bei der Filtereinheit angebrachte Feuchtigkeitssensor. Die Kommunikation mit der Auswerteeinheit kann dabei bspw. über elektrische Kontaktierung mittels der Verbindungselemente erfolgen. Wenn der Feuchtigkeitssensor die Uberschreitung eines vorgegebenen Grenzwertes meldet, wird ein Alarmsignal ausgelöst und/oder die Gasversorgungseinrichtung angehalten. Weiterhin ist es möglich, das Sterilfilterelement als verbraucht zu kennzeichnen (bspw. durch Sperrung des Codeelements in der Auswerteeinheit und/oder Schreiben eines Sperrcodes in das Identelement).

Im Rahmen der Erfindung kann vorgesehen sein, daß eine eventuelle Verstopfung des Sterilfilterelements bzw. der Filtereinheit aufgrund von gasversorgungseitig auftretenden bzw. zum Sterilfilterelement transportierten nichtgasförmigen Verunreinigungen (z.B. mit dem Ergebnis einer störenden Filterkuchenbildung) automatisch erkannt wird und zu einem Warnsignal und/oder zu einem Auswurf des Sterilfilterelements führt in Verbindung mit den vorstehenden Maßnahmen zur Verhinderung einer Wiederbenutzung des Sterilfilterelements. Die automatische Erkennung kann anhand des bei bestimmten Drucken einstellbaren Durchflusses an Gas erfogen, da die Druck/Durchfluß-Verhältnisse von dem Stromungswiderstand des Sterilfilterelements mit bestimmt werden.

Im folgenden wird die Erfindung anhand von lediglich einem Ausführungsbeispiel darstellenden Figuren näher erläutert.
Es zeigen:
- Fig. 1:: Eine Gesamtansicht einer erfindungsgemäßen Vorrichtung,
- Fig. 2:: Eine Detailansicht des Gegenstandes der Figur 1 im Bereich des gasversorgungseinrichtungsseitigen Verbindungselements, und
- Fig. 3:: Eine Detailansicht eines erfindungsgemäß eingesetzten Sterilfilterelements zum Anschluß an den Gegenstand der Fig. 2.

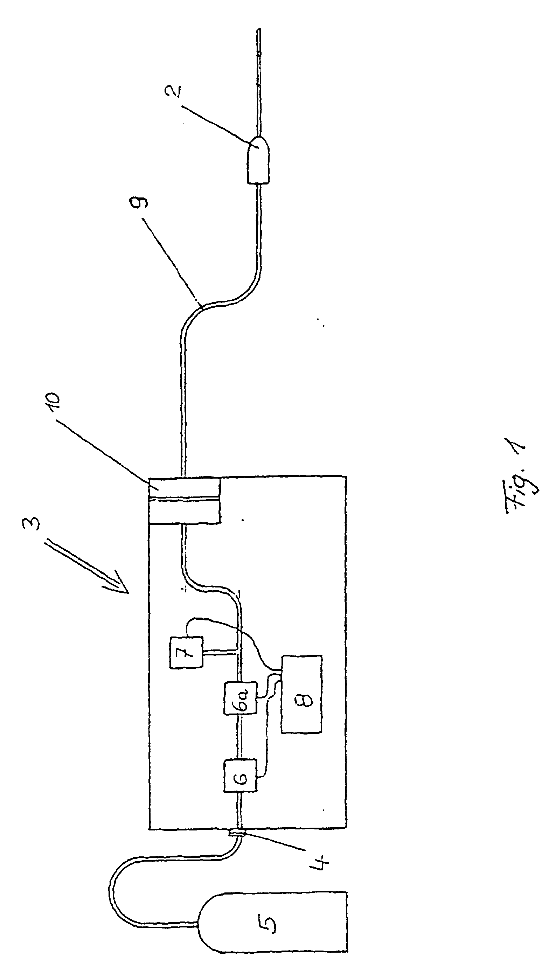

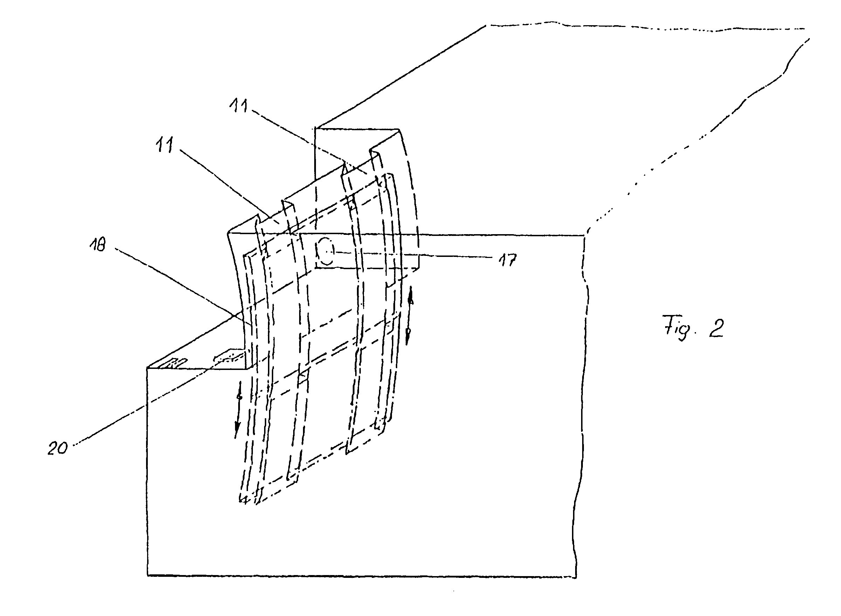

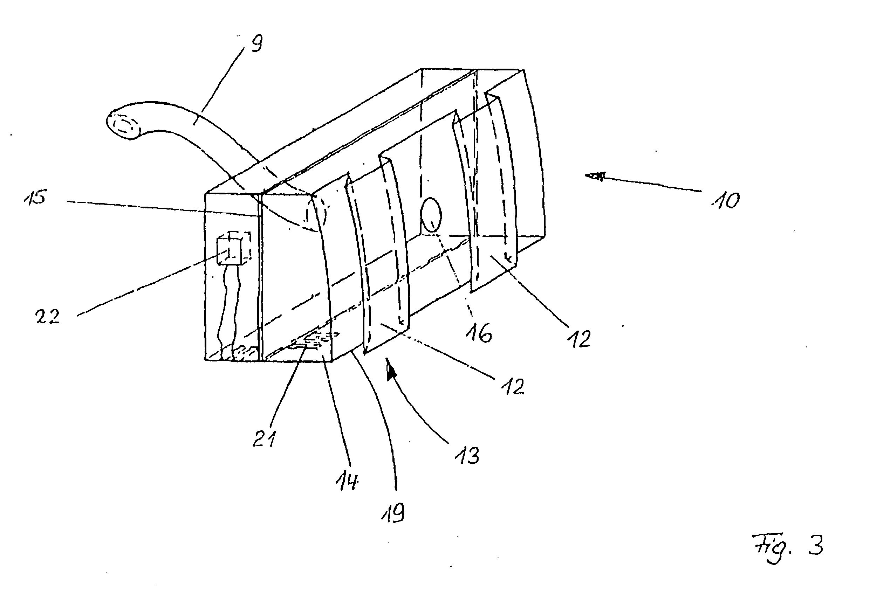

In der Figur 1 erkennt man eine Vorrichtung zum Insufflieren von Gas für die Endoskopie. Eingesetzt wird Kohlendioxid. Als Insufflationsinstrument kommt eine über einen Schlauch 9 mit einer Gasversorgungseinrichtung 3 mittels einer Schlauchanschlußschnittstelle verbindbare Veress-Nadel 2 zum Einsatz, welche im Zuge eines ärztlichen Eingriffs in einen menschlichen Körper eingeführt wird. Die Gasversorgungseinrichtung 3 umfaßt einen Anschluß 4 an eine Kohlendioxidquelle 5, einen Druckminderer 6, einen Durchflußmesser 6a und Druckmesser 7 sowie eine Steuerschaltung 8 zur Druck- bzw. Durchflußsteuerung. Es versteht sich, daß die genannten Bauelemente in geeigneter Weise über Gasleitungen (Schläuche oder Rohre) miteinander verbunden sind. Im Rahmen des Schlauches 9 ist gasversorgungseinrichtungsseitig ein Sterilfilterelement 10 eingerichtet. Die Schlauchanschlußschnittstelle besteht aus zwei zueinander komplementären Verbindungselementen 11, 12, wovon ein Verbindungselement 11 im Bereich des Gehäuses der Gasversorgunseinrichtung 3 und das andere Verbindungselement 12 durch einen Anschlußbereich 13 eines Filtergehäuses 14, welches eine Filtereinheit 15 aufweist, gebildet ist. Hierzu wird ergänzend auf eine vergleichende Betrachtung der Figuren 2 und 3 verwiesen.

In den Figuren 2 und 3 erkennt man, daß die beiden Verbindungselemente 11, 12 als zueinander komplementäre Schwalbenschwanzelemente 11, 12 ausgebildet sind, welche entlang von zwei zueinander parallelen Zylindermantelteilflächen verlaufen. Die beiden Schwalbenschwanzelemente 11, 12 sind ineinander einschiebbar und bilden so in Zugrichtung des Schlauches 9 feste mechanische Verbindung. Es versteht sich, daß das im Bereich der Gasversorgungseinrichtung 3 vorgesehene Schwalbenschwanzelement 11 hierzu jedenfalls von einer Seite entsprechend für das Sterilfilterelement 10 zugänglich sein muß. Im eingeschobenen Zustand kommen ein Sterilfilterelementlumen 16 und ein Gasversorgungseinrichtungslumen 17 zu Deckung mit der Folge, daß Gas in den Schlauch 9 einströmen kann. In der Fig. 2 erkennt nan, daß im Rahmen der Gasversorgungseinrichtung 3 ein Verschlußschieber 18 eingerichtet ist, welcher bei nicht eingeschobenem Sterilfilterelement 10 das Gasversorgungseinrichtungslumen 17 gasdicht abschließt. Nicht dargestellt ist, daß der Verschlußschieber 18 in seine Geschlossenstellung federkraftbeaufschlagt ist, und daß eine elektromechnisch betätigbare Sperrklinke zur Haltung des Verschlußschiebers 18 in seiner Offenstellung eingerichtet ist. Beim Einschieben des Sterilfilterelements 10 wird der Verschlußschieber 18 gegen die Federkraft aus seiner Geschlossenstellung in die Offenstellung verschoben. Dies geschieht mit einer Anschlagflache 19 des Filtergehäuses 14, welche als Betatigungselement fur den Verschlußschieber 18 funktioniert.

In der Figur 3 erkennt man, daß an dem Filtergehause 14 ein Barcode 21 angebracht ist, welcher fur jedes Sterilfilterelement 10 individuell codiert. Durch vergleichende Betrachtung der Figuren 2 und 3 erkennt man, daß dieser Barcode 21 im eingeschobenen Zustand des Sterilfilterelements 10 in dem Bereich einer im Rahmen der Gasversorgungseinrichtung 3 eingerichteten Leseeinheit 20 zum Ruhen kommt und von dieser gelesen werden kann. Der gelesene Barcode 21 wird im Rahmen einer nicht näher dargestellten Auswerteinheit mit zuvor gelesenen und als "verbraucht" abgespeicherten Barcodes verglichen und nur wenn der Vergleich negativ ausfällt, wird die Steuerschaltung 8 freigeschaltet mit dem Ergebnis, daß nur mit einem neuen Sterilfilterelement 10 ein Gasfluß stattfinden kann.

In der Figur 3 erkennt man schließlich, daß ein Feuchtigkeitssensor 22 vorgesehen ist, welcher bei Reflux von Aerosolen oder Körperflüssigkeiten ein Alarmsignal und/oder eine Deaktivierung der Gasversorgungseinrichtung 3 bewirkt.

## Patentansprüche

1. Vorrichtung zum Insufflieren von Gas in einen menschlichen oder tierischen Körper mittels eines über einen Schlauch (9) an eine Gasversorgungseinrichtung (3) anschließbaren Insufflationsinstruments (2),
wobei der Schlauch (9) mit der Gasversorgungseinrichtung (3) mittels einer Schlauchanschlußschnittstelle, aufweisend zueinander komplementäre Verbindungselemente (11, 12) an oder in der Gasversorgungseinrichtung (3) sowie an dem gasversorgungsseitigem Ende des Schlauches (9), verbindbar ist, und
wobei ein Sterilfilterelement (10) eingerichtet ist,
wobei das Sterilfilterelement (10) das Verbindungselement (12) des Schlauches (9) trägt,
**dadurch gekennzeichnet, dass**
der Schlauch (9) mit dem Sterilfilterelement (10) fest verbunden ist und
dass das Sterilfilterelement (10) ein maschinenelesbares Identelement (21) aufweist, und dass das Identelement (21) ein für jedes Sterilfilterelement (10) verschiedenes Codeelement aufweist, damit die Wiederverwendung bereits gebrauchter Sterilfilterelemente verhindert wird.

2. Vorrichtung nach Anspruch 1, **dadurch gekennzeichnet, daß** das Sterilfilterelement (10) ein Filtergehäuse (14) sowie eine darin angeordnete Filtereinheit (15) aufweist, wobei ein Anschlußbereich (13) des Filtergehäuses (14) als Verbindungselement (12) ausgebildet ist.

3. Vorrichtung nach Anspruch 1 oder 2, **dadurch gekennzeichnet, daß** die Verbindungselemente (11, 12) zueinander komplementäre Schwalbenschwanzelemente (11, 12), welche ineinander einschiebbar sind, aufweisen.

4. Vorrichtung nach einem der Ansprüche 1-3, **dadurch gekennzeichnet, daß** zumindest eines der Verbindungselemente (11, 12) ein Betätigungselement für einen in der Gasversorgungseinrichtung (3) eingerichteten Verschlußschieber (18) aufweist, welcher bei nicht angeschlossenem Verbindungselement (12) des Schlauches die Gasversorgungseinrichtung (3) gasdicht abschließt, vorzugsweise ein Gasversorgungseinrichtungslumen (17) im Bereich des Verbindungselements (11) der Gasversorgungseinrichtung (3) gasdicht abschließt.

5. Vorrichtung nach einem der Ansprüche 1-4, **dadurch gekennzeichnet, daß** das Sterilfilterelement (10) ein maschinenlesbares Identelement (21) aufweist, wobei das Identelement (21) mit der Maßgabe angeordnet ist, daß es bei miteinander verbundenen Verbindungselementen (11, 12) mittels einer im Rahmen der Gasversorgungseinrichtung (3) eingerichteten und an eine Auswerteeinheit angeschlossenen Leseeinheit (20) lesbar ist.

6. Vorichtung nach dem Anspruch 5 wenn abhängig von Anspruch 2, **dadurch gekennzeichnet, daß** im Sterilfilterelement (10) abströmungsseitig der Filtereinheit (15) ein Feuchtigkeitssensor (22) angeordnet ist, welcher bei miteinander verbundenen Verbindungselementen (11, 12) zur Kommunikation mit der Auswerteeinheit eingerichtet ist.

## Claims

1. Apparatus for insufflation of gas into a human or animal body by means of an insufflation instrument (2) being connectable with a gas supply (3) by means of a hose (9),
wherein the hose (9) is connectable with the gas supply (3) by means of a hose interface comprising connecting elements (11, 12) at or in the gas supply (3) and at the gas supply end of the hose (9) and being complementary to each other, and
wherein a sterile filter element (10) is provided,
wherein the sterile filter element (10) carries the connecting element (12) of the hose (9),
**characterized in that**
the hose (9) is firmly connected with the sterile filter element (10) and that
the sterile filter element (10) comprises a machine readable identity element (21) and the identity element (21) comprises a code element being different for each sterile filter element (10), for the purpose of preventing re-use of already used sterile filter elements (10).

2. Apparatus according to claim 1, **characterized in that** the sterile filter element (10) comprises a filter housing (14) and a filter unit (15) placed therein,
wherein a connecting area (13) of the filter housing (14) is formed as connecting element (12).

3. Apparatus according to claim 1 or 2, **characterized in that** the connecting elements (11, 12) comprise dovetail elements (11, 12) being complementary to each other and being insertable into each other.

4. Apparatus according to one of the claims 1 - 3, **characterized in that** at least one of the connecting elements (11, 12) comprises an actuator element for a shutter slide (18) provided in the gas supply (3), which shuts off the gas supply (3) in a gas tight manner if the connecting element (12) of the hose (9) is not connected, preferably shuts off in a gas tight manner a gas supply lumen (17) provided in the vicinity of the connecting element (11) of the gas supply (3).

5. Apparatus according to one of the claims 1 - 4, **characterized in that** the sterile filter element (10) comprises a machine readable identity element (21),
wherein the identity element (21) is positioned so that it is readable by means of a read-out unit (20) provided within the gas supply (3) and being connected to an evaluation unit, if the connecting elements (11, 12) are connected with each other.

6. Apparatus according to claim 5, if dependent from claim 2, **characterized in that** a humidity sensor (22) is provided within the sterile filter element (10) and positioned down stream of the filter unit (15), which is capable of communicating with the evaluation unit, if the connecting elements (11, 12) are connected with each other.

## Revendications

1. Dispositif pour insuffler un gaz dans un corps humain ou animal au moyen d'un instrument d'insufflation (2) raccordable par l'intermédiaire d'un tuyau flexible (9) à un dispositif d'alimentation de gaz (3),
le tuyau flexible (9) étant raccordable au dispositif d'alimentation de gaz (3) par l'intermédiaire d'une jonction de raccord de tuyau flexible (9) comprenant des éléments de jonction (11, 12) complémentaires au ou dans le dispositif d'alimentation de gaz (3), et
un élément de filtre stérile (10) étant prévu,
l'élément de filtre stérile (10) portant l'élément de jonction (12) du tuyau flexible (9),
**caractérisé en ce que**
le tuyau flexible (9) est fixé à l'élément de filtre stérile (10), et que
l'élément de filtre stérile (10) comprend un élément d'identification (21) exploitable par une machine, et l'élément d'identification (21) comprend un élément de codage étant différent pour chaque élément de filtre stérile (10), de sorte que la réutilisation d'éléments de filtre stérile qui ont déjà servi soit empêchée.

2. Dispositif selon la revendication 1, **caractérisé en ce que** l'élément de filtre stérile (10) comprend un boîtier de filtre (14) et une unité de filtration (15) disposée là-dedans, une région de raccord (13) du boîtier de filtre (14) étant agencée comme élément de jonction (12).

3. Dispositif selon la revendication 1 ou 2, **caractérisé en ce que** les éléments de jonction (11, 12) comprennent des éléments à queue d'aronde (11, 12) complémentaires coulissant l'un dans l'autre.

4. Dispositif selon une des revendications 1 à 3, **caractérisé en ce qu'**au moins un des éléments de jonction (11, 12) comprend un élément de manoeuvre pour une coulisse de fermeture (18) arrangé dans le dispositif d'alimentation de gaz (3), qui scelle hermétiquement le dispositif d'alimentation de gaz (3) lors d'un élément de jonction (12) non raccordé du tuyau flexible, de préférence un lumen de dispositif d'alimentation de gaz (17) dans la région de l'élément de jonction (11) du dispositif d'alimentation de gaz (3).

5. Dispositif selon une des revendications 1 à 4, **caractérisé en ce que** l'élément de filtre stérile (10) comprend un élément d'identification (21) exploitable par une machine, l'élément d'identification (21) étant agencé de telle manière, qu'il soit exploitable lors des éléments de jonctions (11, 12) raccordés l'un à l'autre au moyen d'une unité lecteur (20) prévue dans le dispositif d'alimentation de gaz (3) et raccordée à une unité d'évaluation.

6. Dispositif selon la revendication 5, si dépendent de la revendication 2, **caractérisé en ce que** dans l'élément de filtre stérile (10) en aval de l'unité de filtration (15), un capteur d'humidité (22) est prévu, qui est agencé lors des éléments de jonction (11, 12) raccordés l'un à l'autre pour la communication avec l'unité d'évaluation.
